# EUROPÄISCHE PATENTSCHRIFT

(11) **EP 0 933 374 B1**
(45) Veröffentlichungstag und Bekanntmachung des Hinweises auf die Patenterteilung: **16.06.2004**
(21) Anmeldenummer: 98122406.6
(22) Anmeldetag: 26.11.1998
(51) Int. Cl.: C07D 309/18, C07D 307/28, B01J 27/043

(54) **Verbessertes Verfahren zur Herstellung von ungesättigten cyclischen Ethern**
Improved method for preparing unsaturated cyclic ethers
Méthode améliorée de préparation d'éthers cycliques insaturés

(30) Priorität: 29.01.1998 DE 19803368
(43) Veröffentlichungstag der Anmeldung: 04.08.1999
(73) Patentinhaber: BASF AKTIENGESELLSCHAFT, 67056 Ludwigshafen (DE)
(72) Erfinder: Gröning, Carsten Dr., 68259 Mannheim (DE); Hesse, Michael Dr., 67549 Worms (DE); Heinecke, Daniel Dr., 67487 Maikammer (DE); Kneuper, Heinz-Josef Dr., 68163 Mannheim (DE); Fritz, Gerhard Dr., 67125 Dannstadt-Schauernheim (DE)

(56) Entgegenhaltungen:
- EP-A- 0 761 662
- US-A- 2 993 910
- US-A- 4 032 475
- CHEMICAL ABSTRACTS, vol. 111, no. 24, 11. Dezember 1989 Columbus, Ohio, US; abstract no. 216885, DEES, M. J. ET AL: "Influence of sulfur and carbonaceous deposits on the selectivity and activity of platinum / cobalt catalysts in hydrocarbon reactions" XP002101317 & J. CATAL. (1989), 119(2), 376-87 CODEN: JCTLA5;ISSN: 0021-9517,

## Beschreibung

Die vorliegende Erfindung betrifft ein Verfahren zur Herstellung von ungesättigten cyclischen Ethern aus Diolen in flüssiger Phase an cobalt- und edelmetallhaltigen Trägerkatalysatoren sowie neue cobalt- und edelmetallhaltige Trägerkatalysatoren, die mit Schwefel dotiert sind.

Aus der DE-A-23 46 943 ist ein Verfahren zur Herstellung ungesättigter cyclischer Verbindungen aus Diolen unter einem Wasserstoffstrom bekannt, in dem als Katalysatoren Gemische aus einem Kupferchromit- oder Kupferträgerkatalysator und einer Wolfram- oder Heteropolywolframsäure eingesetzt werden. Die Umsätze und Ausbeuten lassen zu wünschen übrig.

Aus der US-A-2 993 910 ist ein Verfahren zur Herstellung von Dihydrofuranen aus 1,4-Butandiolen an Cobaltkatalysatoren, die bei 300 bis 450°C mit Wasserstoff reduziert werden müssen, bekannt.

Aus der DE-A-195 30 993 ist ein Verfahren zur Herstellung von ungesättigten cyclischen Ethern an platindotierten, cobalthaltigen Trägerkatalysatoren bekannt.

Aus US 4,032,475 sind halogenierte, mit Platinmetallen datierte Cobalt-zum-Trägerkatalysatoren zur Umsetzung von Kohlenwasserstoffen bekannt, bei denen Cobalt durch eine erst nach einer vorangehenden Reduktion durchgeführte Sulfidierung selektiv in Cobaltsulfid überführt wird.

M.J. Dees et al. J. Catal., 119, 376-87 (1989) beschreiben Kohlenwasserstoff-Umsetzungen an halogenierten Platin-Cobalt-Trägerkatalysatoren, die nach einer vorangehenden Reduktion mit Thiophen-Wasserstoff-Gemischen behandelt werden.

Die bekannten Katalysatoren weisen nicht in allen Anwendungen genügend hohe Aktivitäten und befriedigende Standzeiten des Katalysators auf. Insbesondere ist die Reproduzierbarkeit der Katalysatoraktivität für die Umsetzung von 1,5-Pentandiol zu 3,4-Dihydro-2H-pyran unbefriedigend.

Weiterhin ist gerade für diese Reaktion ein möglichst geringer Gehalt des Nebenproduktes Tetrahydropyran im Reaktionsaustrag für eine technische Verwertung des 3,4-Dihydro-2H-pyrans entscheidend, um aufgrund der dicht beieinanderliegenden Siedepunkte eine wirtschaftliche Reinigung des Reaktionsaustrags zu ermöglichen.

In Anbetracht des Standes der Technik liegt der vorliegenden Erfindung die Aufgabe zugrunde, ein Verfahren zur Herstellung von ungesättigten cyclischen Ethern aus Diolen bereitzustellen, das hohe Katalysatorbelastungen sowie lange Katalysatorstandzeiten bei einer gesteigerten Katalysatoraktivität mit gut reproduzierbaren Katalysatoren ermöglicht und eine möglichst hohe Selektivität aufweist, um die Bildung des Nebenproduktes Tetrahydropyran zurückzudrängen.

Die Aufgabe wird erfindungsgemäß gelöst durch ein neues und verbessertes Verfahren zur Herstellung von ungesättigten cyclischen Ethern der allgemeinen Formel I 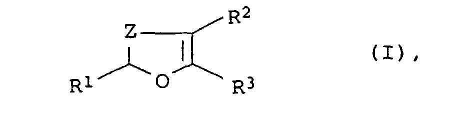 in der
- Z: -(CHR⁴)_{q}- oder - (CHR⁴)_{q}-O-,
- q: 0, 1, 2 oder 3 und
- R¹, R², R³, R⁴: Wasserstoff oder C₁- bis C₄-Alkyl
bedeuten, durch Umsetzung von Diolen der allgemeinen Formel II 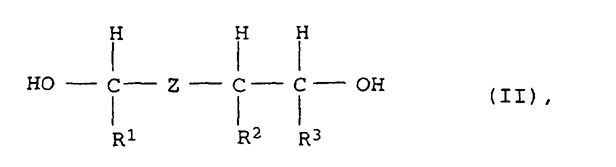 in der Z, R¹, R² und R³ die obengenannten Bedeutungen haben, in flüssiger Phase bei Temperaturen von 150 bis 300°C in Gegenwart eines vor seiner Verwendung nicht durch Reduktion aktivierten, cobalthaltigen Trägerkatalysators, der Cobalt und ein durch Soltränkung aufgebrachtes Edelmetall - aus der Gruppe Platin, Palladium, Rhodium, Iridium, Ruthenium, Osmium, Rhenium oder deren Gemische - auf einem inerten Träger enthält, dadurch gekennzeichnet, daß der Trägerkatalysator mit Schwefel dotiert ist.

Die Substituenten R¹, R², R³, R⁴, das Zwischenglied Z und der Index q in den Verbindungen I und II haben folgende Bedeutungen:
- R¹, R², R³, R⁴: unabhängig voneinander
- Wasserstoff,
- C₁- bis C₄-Alkyl wie Methyl, Ethyl, n-Propyl, iso-Propyl, n-Butyl, iso-Butyl, sec.-Butyl und tert.-Butyl, bevorzugt C₁- bis C₃-Alkyl wie Methyl, Ethyl, n-Propyl und iso-Propyl, besonders bevorzugt Methyl und Ethyl,
- Z: -(CHR⁴)_{q}- oder -(CHR⁴)_{q}-O-,
- q: 0, 1, 2 oder 3, bevorzugt 0 und 1, besonders bevorzugt 1.

Als Diole II eignen sich z.B. 1,4-Butandiol, 1,5-Pentandiol, 1,6-Hexandiol, Diethylenglykol, bevorzugt 1,5-Pentandiol.

Als ungesättigte cyclische Ether I eignen sich z.B. 3,4-Dihydro-2H-pyrane, 2,3-Dihydrofuran und 1,4-Dioxan, bevorzugt 3,4-Dihydro-2H-pyran.

Das erfindungsgemäße Verfahren läßt sich wie folgt durchführen:

Man kann das Diol II in der Regel mit 0,2 bis 20 Gew.-%, bevorzugt 0,3 bis 10 Gew.-% cobalthaltigem Trägerkatalysator bei Temperaturen von 150 bis 300°C, bevorzugt 160 bis 240°C umsetzen. Der cobalthaltige Trägerkatalysator kann vorgelegt werden oder während der Reaktion gestuft in Anteilen von der Gesamtmenge zugegeben werden. Das Reaktionsgemisch sollte während der Umsetzung gleichmäßig gerührt werden, wobei die Drehzahl des Rührwerks so zu wählen ist, daß der Katalysator durch die eingetragene Rührenergie mechanisch nicht zu sehr belastet wird. Das entstandene Gemisch aus dem ungesättigten cyclischen Ether I und dem Reaktionswasser kann diskontinuierlich, bevorzugt kontinuierlich abdestilliert werden. Der bei der Reaktion entstehende ungesättigte cyclische Ether kann gegebenenfalls zur Entfernung des bei der Reaktion entstehenden Wasserstoffs mit unter Reaktionsbedingungen inerten Gasen, wie Stickstoff oder Argon, gestrippt werden. Bei kontinuierlicher Fahrweise kann durch Zufuhr von frischem Diol II der Flüssigkeitsstand im Reaktionsgefäß gehalten werden. Der Zusatz von Alkali- und/oder Erdalkalimetallverbindungen, um den Gehalt an destillativ nur schwer vom ungesättigten 3,4-Dihydro-2H-pyran abtrennbaren gesättigten cyclischen Ether zu senken, ist bei dem erfindungsgemäßen Verfahren nicht erforderlich.

Als cobalt- und edelmetallhaltige, mit Schwefel dotierte Trägerkatalysatoren eignen sich die Oxide des Cobalts oder metallisches Cobalt und ein oder mehrere Elemente der Edelmetalle aus der Gruppe Platin, Palladium, Rhodium, Iridium, Ruthenium, Osmium, Rhenium oder deren Gemische, bevorzugt Platin, Palladium, Rhenium oder deren Gemische, besonders bevorzugt Platin, Palladium oder deren Gemische sowie gegebenenfalls 0,001 bis 10 Gew.-%, bevorzugt 0,1 bis 5 Gew.-%, besonders bevorzugt 0,5 bis 3 Gew.-% basische Alkali-, Erdalkalimetallsalze, Scandium, Vanadin, Chrom, Mangan, Eisen, Nickel, Kupfer, Zink, Germanium, Zinn, Blei, Antimon, Wismut oder deren Gemische (Verbindung A), bevorzugt Lithium, Kalium, Natrium, Calcium, Strontium, Barium, Mangan, Eisen, Nickel, Kupfer, Zink, Zinn, Antimon oder deren Gemische, besonders bevorzugt Kalium, Natrium, Mangan, Eisen, Nickel, Kupfer, Zink oder deren Gemische auf einem porösen Träger.

Der Gewichtsanteil des Cobalt(-oxides) im Trägerkatalysator beträgt in der Regel 1 bis 70 Gew.-%, bevorzugt 5 bis 50 Gew.-%, besonders bevorzugt 10 bis 40 Gew.-%.

Der Gewichtsanteil der Elemente aus der Gruppe Platin, Palladium Rhodium, Iridium, Ruthenium, Osmium oder Rhenium, bevorzugt Platin, Palladium oder Rhenium, beträgt 0,001 bis 2 Gew.-%, bevorzugt 0,05 und 1 Gew.-%, besonders bevorzugt 0,01 bis 0,5 Gew.-%, bezogen auf den Trägerkatalysator.

Der Gewichtsanteil des Schwefels (berechnet als S) liegt zwischen 0,015 und 2 Gew.-%, bevorzugt 0,1 bis 1 Gew.-%, insbesondere bevorzugt 0,3 bis 0,6 Gew.-%, bezogen auf den Trägerkatalysator

Zur Bestimmung des Schwefelgehalts der Katalysatoren wird der Trägerkatalysator in Salzsäure gelöst und mit hypophosphoriger Säure behandelt. Der dabei gebildete Schwefelwasserstoff wird im N₂-Strom ausgetrieben, in ammoniakalischer Cadmiumacetatlösung aufgefangen und jodometrisch bestimmt.

Die Trägerkatalysatoren weisen in der Regel ein Gewichtsverhältnis von Cobalt(-oxid) zum Edelmetall von 10 : 1 bis 10.000 : 1 und von Edelmetall zum Schwefel von 100 : 1 bis 1 : 100 auf.

Als Träger eignen sich inerte Träger wie SiO₂, Al₂O₃, TiO₂, ZrO₂, Zeolithe aller Art wie kleinporige Zeolithe, z.B. A-Zeolith, mittelporige Zeolithe, z.B. ZSM-5, ZSM-11, Ferrierit, großporige Zeolithe, z.B. Faujasite, β-Zeolithe, Mordenit, Offretit, hydrothermal hergestellte Phosphate wie AlPO und SAPO, Aktivkohlen oder Erdalkalioxide, bevorzugt SiO₂, ZrO₂ und Zeolithe, besonders bevorzugt SiO₂, wobei in der Regel ein Gewichtsverhältnis von Cobalt(-oxides) zum SiO₂ im Trägerkatalysator 1 : 20 bis 1 : 1 vorliegt.

Die Trägerkatalysatoren weisen in der Regel eine BET-Oberfläche von 1 bis 600 m²/g, bevorzugt 10 bis 500 m²/g, besonders bevorzugt 50 bis 400 m²/g auf.

Die Porosität der Trägerkatalysatoren liegt in der Regel bei 0,01 bis 1,5 ml/g, bevorzugt 0,1 bis 1,2 ml/g, besonders bevorzugt 0,2 bis 1 ml/g.

Die erfindungsgemäß verwendeten Trägerkatalysatoren werden hergestellt, indem man zunächst Cobalt, dann das Edelmetall in Form eines Sols auf den Träger aufbringt und anschließend mit Schwefel dotiert.

Die Herstellung der cobalthaltigen Trägerkatalysatoren ist allgemein bekannt. Vorteilhaft ist die Tränkung des porösen Trägermateriales mit einer löslichen Cobaltverbindung (z.B. eines Nitrits, Nitrats, Sulfits, Sulfats, Carbonats, Hydroxids, von Carboxylaten, Halogeniden, Halogeniten, Halogenaten u.a.), gegebenenfalls gleichzeitig oder nacheinander mit einer ebenfalls löslichen Verbindung A (z.B. als Nitrit, Nitrat, Sulfit, Sulfat, Carbonat, Hydroxid, Carboxylate, Halogenide, Halogenite, Halogenate u.a.), und anschließender thermischer Zersetzung des Anions zum Oxid. Eine weitere Möglichkeit ist das Mischen einer Cobalt-Verbindung mit dem Trägermaterial (trocken oder in Suspension, insbesondere durch Sprühtrocknung), gegebenenfalls gleichzeitig mit einer chemischen Verbindung A, Verdichtung des Materials (z.B. durch Verkneten, gegebenenfalls Zugabe eines geeigneten Verformungshilfsmittels), Formgebung durch Extrudieren, Trocknung und anschließender Calcinierung bei Temperaturen von 200 bis 1300°C, bevorzugt 300 bis 1000°C, besonders bevorzugt 400 bis 800°C.

Das Edelmetall - aus der Gruppe Platin, Palladium, Rhodium, Iridium, Ruthenium, Osmium, Rhenium oder deren Gemische - wird sodann durch das Besprühen des noch heißen Trägers oder durch Tränkung des Trägers mit einem zuvor hergestellten Sol auf den Träger aufgebracht, wobei zuvor gegebenenfalls durch die vorangegangene Tränkung agglomerierte Katalysatormasse zerkleinert wird.

Das Edelmetallsol ist ein kolloidaler Stoff und kann nach bekannten Methoden, z.B. ausgehend von Metallsalzen, in denen das Edelmetall in einer Oxidationsstufe größer Null vorliegt, hergestellt werden. Es können z.B. wäßrige Lösungen der Chloride, Acetate oder Nitrate des Metalls verwendet werden. Es sind aber auch andere Edelmetallsalze verwendbar; eine Beschränkung hinsichtlich des Anions besteht nicht. Als Reduktionsmittel lassen sich organische Verbindungen wie Ethanol, Methanol, Carbonsäuren und deren Alkalisalze sowie anorganische Verbindungen wie N₂H₄ oder NaBH₄ verwenden. Bevorzugt sind Hydrazin N₂H₄ und Ethanol. Die Teilchengröße der Metallpartikel im Sol hängt hierbei von der Stärke des eingesetzten Reduktionsmittels und vom verwendeten Metallsalz ab. Die Sole lassen sich durch Zugabe von organischen Polymeren wie Polyaminen, Polyvinylpyrrolidon oder Polyacrylaten stabilisieren, wobei Polyvinylpyrrolidon PVP bevorzugt ist, Die Solherstellung kann aber auch nach anderen in der Literatur beschriebenen Vorgehensweisen durchgeführt werden. Bönnemann et al. (Angew. Chemie, 103 (1991), 1344) beschreiben beispielsweise die Herstellung von stabilen Metallsolen durch Reduktion von Metallsalzen mit (C₈H₁₇)₄N[BEtH₃].

Die Aufbringung der Sole auf den Träger kann nach verschiedenen Techniken erfolgen, die die Verteilung der Aktivkomponente beeinflussen. Zur Erzeugung dünner Schalen der Aktivkomponente für den gesamten Strangquerschnitt wird das Sol auf einen indirekt beheizten Träger aufgesprüht. Man geht dabei so vor, daß der Träger in einem drehbaren, beheizbaren Pelletierteller vorgelegt und durch ein Heißluftgebläse auf Temperaturen zwischen 80 und 200°C aufgeheizt wird. Während der Teller gedreht wird, sprüht man das Sol auf den Träger auf. Durch das Drehen des Tellers wird die Durchmischung der Trägerteilchen, z.B. Stränge oder Splitt, gewährleistet. Beim Kontakt mit dem heißen Träger verdampft die Flüssigkeit im Sol und die Aktivkomponente bleibt auf dem Träger zurück. Durch diese Aufbringtechnik entstehen Katalysatoren, in denen die Aktivkomponente in dünnen Schichten, die im allgemeinen kleiner als 50 µm sind, auf dem Träger aufgebracht ist. Die Teilchengröße der Edelmetallagglomerate liegt im allgemeinen in derselben Größenordnung wie im Sol. Der Katalysator wird dann bei einer Temperatur, die 150°C nicht überschreitet, getrocknet.

Eine andere Technik zur Aufbringung der Aktivkomponente besteht darin, den Träger entsprechend seiner vorher ermittelten Wasseraufnahme, die im wesentlichen seinem Porenvolumen entspricht, mit einem Metallsol zu tränken. Nach Abtropfen des Trägers wird dieser dann bei einer Temperatur, die 150°C nicht übersteigt, getrocknet. Derart hergestellte Katalysatoren weisen die Aktivkomponente überraschenderweise ebenfalls in einer sehr dünnen Schicht auf. In diesem Fall liegt die Aktivkomponente jedoch, wenn makroporöse Träger eingesetzt werden, in den von außen zugänglichen Makroporen angereichert vor, während bei Aufsprühen des Sols im wesentlichen eine Gleichverteilung der Aktivkomponente in Mikro- und Makroporen erfolgt.

In einem dritten Schritt der Katalysatorherstellung wird anschließend der cobalt- und edelmetallhaltige Trägerkatalysator mit Schwefel dotiert, wobei gegebenenfalls durch die vorangegangene Soltränkung agglomeriertes Katalysatormaterial zuvor zerkleinert wird. Die Dotierung des Trägerkatalysators mit Schwefel erfolgt durch Tränkung des Trägers mit einer mindestens eine anorganische und/oder organische Schwefelverbindungen formal negativer Oxidationsstufe des Schwefels, beispielsweise der formalen negativen Oxidationsstufe (-2) des Schwefels enthaltenden wäßrigen oder wasserhaltigen Lösung oder einer Lösung in einem organischen Lösungsmittel, wobei die Verwendung einer wäßrigen Lösung bevorzugt ist. Die wasserhaltigen Lösungen können neben Wasser zusätzlich organische Lösungsmittel enthalten. Als organische Lösungsmittel sind polar protische oder aprotische organische Lösungsmittel, beispielsweise Alkohole wie Methanol geeignet.

Von den zur Schwefeldotierung der erfindungsgemäß verwendeten Katalysatoren geeigneten Schwefelverbindungen mit formal negativer Oxidationsstufe sind Ammoniumsulfid, Thioharnstoff und Mercaptopyrimidin, zum Beispiel 4-Amino-2-mercaptopyrimidin, bevorzugt.

Die Tränkung kann durch Behandeln des Trägermaterials mit einer überstehenden Lösung der Schwefelverbindung bewerkstelligt werden, besonders vorteilhaft erfolgt sie durch Zugabe der die Schwefelverbindung enthaltenden Lösung zum Träger in einer rotierenden Trommel, wobei vorteilhaft eine Menge an Lösung eingesetzt wird, die dem Porenvolumen des Trägers entspricht.

Nach Trocknung und gegebenenfalls Calcinierung, in der Regel bei 250 bis 500°C, vorzugsweise bei 300 bis 400°C, kann der Katalysator im erfindungsgemäßen Verfahren eingesetzt werden.

Es ist vorteilhaft, daß die Katalysatoren vor ihrer Anwendung im erfindungsgemäßen Verfahren nicht durch die Behandlung mit Wasserstoff oder anderen Reduktionsmitteln, wie Hydrazin, aktiviert werden müssen.

Die ungesättigten cyclischen Ether I sind wertvolle Schutzgruppen für Alkohole.

### Beispiele

### Herstellung der Katalysatoren A und B

16,7 l einer Lösung aus 12,61 kg Co(NO₃)₂ · 6 H₂O (entsprechend 3,25 kg CoO) in Wasser wurde mit 10 kg SiO₂-Pulver (Wasseraufnahme 1,85 ml/g) ca. 2 h verrührt, 16 h bei 120°C getrocknet und 2 h bei 500°C calciniert.

Diese Masse wurde sodann mit 15,8 l eines Edelmetall-Sols, hergestellt durch Mischen von 22,3 g Platinnitrat in 4,5 l destilliertem Wasser mit 32 g Polyvinylpyrrolidon und 1,93 l Ethanol, 4 h Erhitzen unter Rückfluß und Verdünnen mit Wasser, getränkt, anschließend bei 100°C im Vakuum getrocknet und 2 h bei 500°C unter N₂-Atmosphäre calciniert. Der so hergestellte Katalysator enthält 0,12 Gew.-% PtO₂.

4,6 g dieser cobalt- und platinhaltigen Massen wurden sodann mit einer Lösung von (NH₄)₂S in Wasser getränkt, anschließend bei 100°C im Vakuum getrocknet und 2 h bei 350°C calciniert.

Weitere Einzelheiten zur Herstellung und zu den Eigenschaften der Katalysatoren A und B sind Tabelle 1 zu entnehmen.

**Tabelle 1**

| Katalysator | Menge Edelmetall-Sol [ml] | Edelmetallgehalt [Gew.-%] | (NH₄)₂S [g] | Schwefelgehalt [Gew.-%] |
|---|---|---|---|---|
| A | 15800 | 0,10 | 0,04 | 0,34 |
| B | 15800 | 0,10 | 0,06 | 0,38 |

### Herstellung des Katalysators C

3400 ml einer Lösung aus 3,25 kg Co(NO₃)₂ · 6 H₂O in Wasser wurde mit 2,5 kg SiO₂-Pulver (Wasseraufnahme 1,5 ml/g) ca. 2 h verrührt, 16 h bei 120°C getrocknet und 2 h bei 500°C calciniert.

Diese Masse wurde sodann mit 2,9 l eines Edelmetall-Sols, hergestellt durch Mischen von 14,2 g Platinnitrat in 4,5 l destilliertem Wasser mit 32 g Polyvinylpyrrolidon und 1,93 l Ethanol und 4 h Erhitzen unter Rückfluß, getränkt, anschließend bei 100°C im Vakuum getrocknet und 2 h bei 500°C unter N₂-Atomosphäre calciniert. Der so hergestellte Katalysator enthält 0,12 Gew.-% PtO₂.

4,6 g dieser cobalt- und platinhaltigen Masse wurde sodann mit einer Lösung von (NH₄)₂S in Wasser getränkt, anschließend bei 100°C im Vakuum getrocknet und 2 h bei 350°C calciniert.

Weitere Einzelheiten zur Herstellung und zu den Eigenschaften des Katalysatos C sind Tabelle 2 zu entnehmen.

**Tabelle 2**

| Katalysator | Menge Edelmetall-Sol [ml] | Edelmetallgehalt [Gew.%] | (NH₄)₂S [g] | Schwefelgehalt [Gew.-%] |
|---|---|---|---|---|
| C | 2900 | 0,10 | 0,04 | 0,34 |

Vergleichskatalysatoren VA und VB (nach DE 19530993) 555 ml einer Lösung aus 291,3 g Co(NO₃)₂ · 6 H₂O und einem Metallnitrat in Wasser wurde mit 300 g SiO₂-Pulver (Wasseraufnahme 1,85 ml/g) ca. 2 h verrührt, 16 h bei 120°C getrocknet und 2 h bei 500°C calciniert. Auf einer aus Tabelle 1 ersichtlichen Menge dieser Masse wurde innerhalb von 2 h ein Edelmetall-Sols (0,6 g/l), hergestellt durch Mischen eines Edelmetallsalzes in 700 ml Wasser mit 5 g Polyvinylpyrrolidon und 300 ml Ethanol und 4 h Erhitzen unter Rückfluß, aufgesprüht, anschließend getrocknet und 1 h bei 500°C calciniert.

Weitere Einzelheiten zur Herstellung und zu den Eigenschaften der Katalysatoren sind Tabelle 3 zu entnehmen:

**Tabelle 3**

| Katalysator | Edelmetallsalz [g] | Menge Co-halt. SiO₂ [g] | Menge Edelmetall-Sol [ml] | Edelmetallgehalt [Gew.-%] | Schwefelgehalt [Gew.-%] |
|---|---|---|---|---|---|
| VA | 1,46 g Pt(NO₃)₂ | 310 | 260 | 0,05 | - |
| VB | 1,46 g Pt (NO₃)₂ | 328,6 | 548 | 0,1 | - |

### Beispiele 1 bis 8

1,5 l 1,5-Pentandiol und 45 g eines Katalysators A, B, VA und VB wurden vorgelegt und unter Rühren bis zur unteren Grenze des Temperaturintervalls erwärmt, wobei die Reaktion unter Wasserstoffentwicklung anspringt. Das entstehende 3,4-Dihydro-2H-pyran/Wasser-Gemisch wurde kontinuierlich abdestilliert und die Temperatur im Sumpf wurde während der Reaktion so nachgeregelt, daß die pro Stunde anfallende Destillatmenge konstant blieb (40 - 50 ml). Gleichzeitig wurde innerhalb der aus Tabelle 4 ersichtlichen Zeiten 1,5-Pentandiol kontinuierlich zudosiert, um den Stand im Reaktionskolben konstant zu halten. Nach Phasentrennung des Destillats erhielt man 3,4-Dihydro-2H-pyran in den in Tabelle 4 angegebenen Mengen.

**Tabelle 4**

| Bsp. | Katalysator | Temperatur [°C] | Dauer [h] | Ausbeute DHP [Gew.-%] | Reinheit DHP | THP Gehalt | Katalysator-aktivität [kg DHP/kg Kat] |
|---|---|---|---|---|---|---|---|
| 1 | A | 170-197 | 200 | 98 | 96 | 1,0 | |
| 2 | A | 170-235 | 780 | 96 | 96 | 0,9 | 534 |
| 3 | B | 170-195 | 200 | 95 | 95 | 1,4 | |
| 4 | B | 170-235 | 740 | 96 | 95 | 1,2 | 525 |
| 5 | VA | 170-212 | 200 | 98 | 97 | 1,5 | |
| 6 | VA | 170-235 | 410 | 98 | 96 | 2,1 | 351 |
| 7 | VB | 170-212 | 200 | 98 | 96 | 1,3 | |
| 8 | VB | 170-235 | 420 | 98 | 95 | 2,0 | 367 |
| DHP = 3,4-Dihydro-2H-pyran | | | | | | | |
| THP = Tetrahydropyran | | | | | | | |

Die in Tabelle 4 zusammengestellten Versuchsergebnisse zeigen deutlich, daß mit den erfindungsgemäß verwendeten Katalysatoren A und B der Anfall an Tetrahydropyran im Austrag deutlich gesenkt werden konnte. Die Katalysatorstandzeit der Katalysatoren A und B ist zudem, wie auch die langen Laufzeiten über 200 h zeigen, wesentlich höher als bei den Vergleichskatalysatoren.

Darüber hinaus zeichnen sich die erfindungsgemäßen Katalysatoren A und B durch eine gute Reproduzierbarkeit aus.

### Beispiel 9

1,5 l 1,5-Pentandiol und b g Katalysator C wurden vorgelegt und unter Rühren bis zur unteren Grenze des Temperaturintervalls von 170 bis 180°C erwärmt, wobei die Reaktion unter Wasserstoffentwicklung ausspringt. Das entstehende 3,4-Dihydro-2H-pyron/Wasser-Gemisch wurde kontinuierlich abdestilliert und die Temperatur im Sumpf wurde während der Reaktion so nachgeregelt, daß die pro Stunde anfallende Destillatmenge konstant blieb (40 - 50 ml). Gleichzeitig wurde innerhalb der aus Tabelle 5 ersichtlichen Zeiten 1,5-Pentandiol kontinuierlich zudosiert, um den Stand im Reaktionskolben konstant zu halten. Tabelle 5 sind die Einzelheiten der Versuchsdurchführung zu entnehmen.

| Katalysator-aktiviät (kg DHP/kg/ Kat) | Dauer [h] | Katalysator-belastung [g DHP/kg Kat/h] | Raum-Zeit-Ausbeute b(g) | Katalysatormenge b(g) | Katalysatorkonzentration |
|---|---|---|---|---|---|
| 567 | 410 | 1383 | 20,7 | 22,5 | 1,478 |
| 800 | 290 | 2759 | 20,7 | 11,25 | 0,744 |
| 834 | 130 | 6415 | 21,4 | 5,00 | 0,322 |

## Patentansprüche

1. Verfahren zur Herstellung von ungesättigten cyclischen Ethern der allgemeinen Formel I 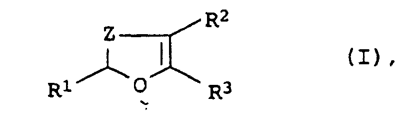 in der
Z -(CHR⁴)_{q}- oder -(CHR⁴)_{q}-O-,
q 0, 1, 2 oder 3 und
R¹,R²,R³,R⁴ Wasserstoff oder C₁- bis C₄-Alkyl
bedeuten, durch Umsetzung von Diolen der allgemeinen Formel II 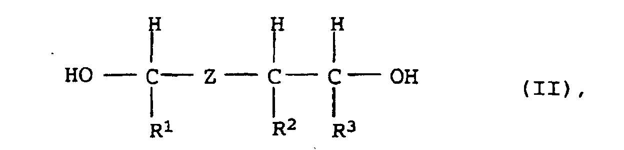 in der Z, R¹, R² und R³ die obengenannten Bedeutungen haben, in flüssiger Phase bei Temperaturen von 150 bis 300°C in Gegenwart eines vor seiner Verwendung nicht durch Reduktion aktivierten, cobalthaltigen Trägerkatalysators, der Cobalt und ein durch Soltränkung aufgebrachtes Edelmetall - aus der Gruppe Platin, Palladium, Rhodium, Iridium, Ruthenium, Osmium, Rhenium oder deren Gemische - auf einem inerten Träger enthält, **dadurch gekennzeichnet, daß** der Trägerkatalysator mit Schwefel dotiert ist.

2. Verfahren zur Herstellung von ungesättigten cyclischen Ethern nach Anspruch 1, **dadurch gekennzeichnet, daß** die cobalthaltigen Trägerkatalysatoren 1 bis 70 Gew.-% Cobalt, 0,001 bis 2 Gew.-% eines oder mehrerer Edelmetalle und 0,015 bis 2 Gew.-% Schwefel enthalten.

3. Verfahren zur Herstellung von ungesättigten cyclischen Ethern nach Anspruch 1 oder 2, **dadurch gekennzeichnet, daß** der Schwefel durch Tränken des Katalysators mit einer eine anorganische oder organische Schwefelverbindungen mit formal negativer Oxidationsstufe des Schwefels oder von deren Gemischen enthaltenden wäßrigen oder wasserhaltigen Lösung oder einer Lösung in einem organischen Lösungsmittel aufgebracht wird.

4. Verfahren zur Herstellung von ungesättigten cyclischen Ethern I nach den Ansprüchen 1 bis 3, **dadurch gekennzeichnet, daß** die cobalthaltigen Trägerkatalysatoren 0,001 bis 10 Gew.-% basische Alkali-, Erdalkalimetallsalze oder deren Gemische bezogen auf den Gesamtmetallgehalt enthalten.

5. Verfahren zur Herstellung von ungesättigten cyclischen Ethern I nach den Ansprüchen 1 bis 4, **dadurch gekennzeichnet, daß** die cobalthaltigen Trägerkatalysatoren 0,001 bis 10 Gew.-% Scandium, Vanadin, Chrom, Mangan, Eisen, Nickel, Kupfer, Zink, Germanium, Zinn, Blei, Antimon, Wismut oder deren Gemische bezogen auf den Gesamtmetallgehalt enthalten.

6. Verfahren zur Herstellung von ungesättigten cyclischen Ethern nach den Ansprüchen 1 bis 5, **dadurch gekennzeichnet, daß** 1,5-Pentandiol zu 3,4-Dihydro-2H-pyran umgesetzt wird.

## Claims

1. A process for preparing unsaturated cyclic ethers of the formula I 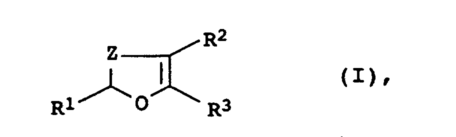 where
Z is -(CHR⁴)_{q}- or -(CHR⁴)_{q}-O-,
q is 0, 1, 2 or 3 and
R¹,R²,R³,R⁴ are hydrogen or C₁-C₄-alkyl,
by reacting diols of the formula II 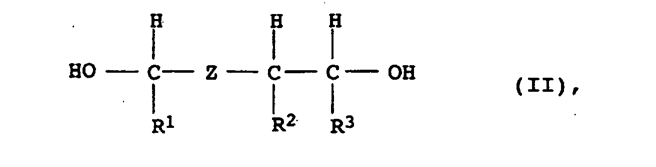 where Z, R¹, R² and R³ are as defined above, in the liquid phase at from 150 to 300°C in the presence of a cobalt-containing supported catalyst which has not been activated by reduction before use and comprises cobalt and a noble metal selected from the group consisting of platinum, palladium, rhodium, iridium, ruthenium, osmium, rhenium or a mixture thereof applied by sol impregnation on an inert support, wherein the supported catalyst is doped with sulfur.

2. A process for preparing unsaturated cyclic ethers as claimed in claim 1, wherein the cobalt-containing supported catalyst comprises from 1 to 70% by weight of cobalt, from 0.001 to 2% by weight of one or more noble metals and from 0.015 to 2% by weight of sulfur.

3. A process for preparing unsaturated cyclic ethers as claimed in claim 1 or 2, wherein the sulfur is applied by impregnating the catalyst with an aqueous or water-containing solution comprising an inorganic or organic sulfur compound having a formally negative oxidation state of the sulfur or a mixture thereof or a solution in an organic solvent.

4. A process for preparing unsaturated cyclic ethers I as claimed in any of claims 1 to 3, wherein the cobalt-containing supported catalyst comprises from 0.001 to 10% by weight of a basic alkali metal salt or alkaline earth metal salt or a mixture thereof, based on the total metal content.

5. A process for preparing unsaturated cyclic ethers I as claimed in any of claims 1 to 4, wherein the cobalt-containing supported catalyst comprises from 0.001 to 10% by weight of scandium, vanadium, chromium, manganese, iron, nickel, copper, zinc, germanium, tin, lead, antimony, bismuth or a mixture thereof, based on the total metal content.

6. A process for preparing unsaturated cyclic ethers as claimed in any of claims 1 to 5, wherein 1,5-pentanediol is converted into 3,4-dihydro-2H-pyran.

## Revendications

1. Procédé de préparation d'éthers cycliques insaturés de formule générale I 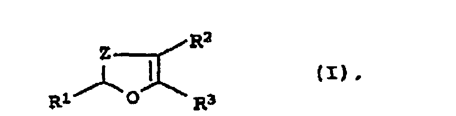 dans laquelle
Z représente -(CHR⁴)_{q}- ou - (CHR⁴)_{q}-O-,
q vaut 0, 1, 2 ou 3 et
R¹, R², R³, R⁴ représentent un atome d'hydrogène ou un groupe alkyle en C₁ à C₄
au moyen de la réaction de diols de formule générale II 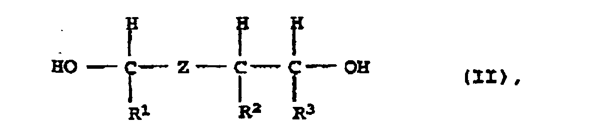 dans laquelle Z, R¹, R² et R³ ont les significations susmentionnée, en phase liquide à des températures allant de 150°C à 300°C en présence d'un catalyseur supporté contenant du cobalt qui n'a pas été activé par une réduction avant son utilisation, qui contient du cobalt et un métal noble - du groupe formé par le platine, le palladium, le rhodium, l'iridium, le ruthénium, l'osmium, le rhénium ou leurs mélanges - déposé par imprégnation sol-gel sur un support inerte, **caractérisé en ce que** le catalyseur supporté est dopé au soufre.

2. Procédé de préparation d'éthers cycliques insaturés selon la revendication 1, **caractérisé en ce que** les catalyseurs supportés contenant du cobalt contiennent 1% à 70% en poids de cobalt, 0,001% à 2% en poids d'un ou de plusieurs métaux nobles et 0,015% à 2% en poids de soufre.

3. Procédé de préparation d'éthers cycliques insaturés selon la revendication 1 ou 2, **caractérisé en ce que** le soufre est déposé au moyen d'une imprégnation du catalyseur avec une solution aqueuse ou hydratée, contenant un composé inorganique ou des composés organiques du soufre ayant un degré d'oxydation formellement négatif du soufre ou leurs mélanges, ou avec une solution dans un solvant organique.

4. Procédé de préparation d'éthers cycliques insaturés I selon les revendications 1 à 3, **caractérisé en ce que** les catalyseurs supportés contenant du cobalt contiennent 0,001% à 10% en poids de sels basiques de métaux alcalins ou alcalino-terreux ou leurs mélanges par rapport à la teneur totale en métal.

5. Procédé de préparation d'éthers cycliques insaturés I selon les revendications 1 à 4, **caractérisé en ce que** les catalyseurs supportés contenant du cobalt contiennent 0,001% à 10% en poids de scandium, de vanadium, de chrome, de manganèse, de fer, de nickel, de cuivre, de zinc, de germanium, d'étain, de plomb, d'antimoine, de bismuth ou de leurs mélanges par rapport à la teneur totale en métal.

6. Procédé de préparation d'éthers cycliques insaturés selon les revendications 1 à 5, **caractérisé en ce que** l'on fait réagir le 1,5-pentanediol pour former le 3,4-dihydro-2H-pyranne.
